Europäisches Patentamt

European Patent Office

Office européen des brevets

(11) Veröffentlichungsnummer: **0 514 968 A1**

## (12) EUROPÄISCHE PATENTANMELDUNG

(21) Anmeldenummer: **92201326.3**

(22) Anmeldetag: **11.05.92**

(51) Int. Cl.5: **G01T 1/29, A61B 6/03**

(30) Priorität: **18.05.91 DE 4116381**

(43) Veröffentlichungstag der Anmeldung:
**25.11.92 Patentblatt 92/48**

(84) Benannte Vertragsstaaten:
**DE FR GB**

(71) Anmelder: **Philips Patentverwaltung GmbH**
**Wendenstrasse 35 Postfach 10 51 49**
**W-2000 Hamburg 1(DE)**

(84) **DE**

(71) Anmelder: **N.V. Philips' Gloeilampenfabrieken**

**Groenewoudseweg 1**
**NL-5621 BA Eindhoven(NL)**

(84) **FR GB**

(72) Erfinder: **Kuhn, Michael, Dr.**
**Süntelstrasse 77**
**W-2000 Hamburg 61(DE)**

(74) Vertreter: **Hartmann, Heinrich, Dipl.-Ing. et al**
**Philips Patentverwaltung GmbH**
**Wendenstrasse 35 Postfach 10 51 49**
**W-2000 Hamburg 1(DE)**

(54) **Computertomograph.**

(57) Die Erfindung betrifft einen Computertomographen für kontinuierliche Rotation.

Ein derartiger Computertomograph kann - ohne Verlängerung der Rekonstruktionszeiten - dadurch vereinfacht werden, daß die Mittel zur Rekonstruktion (11..14) auf den Rotor (4) angeordnet sind und daß eine Übertragungseinrichtung (15,16) vorgesehen ist, die bei Stillstand des Rotors (4) die die Absorptionsverteilung repräsentierenden Daten vom Rotor (4) zum Stator (7) überträgt.

EP 0 514 968 A1

Die Erfindung betrifft einen Computertomographen für kontinuierliche Rotation mit

- einem einen Untersuchungsbereich umschließenden Rotor,
- einem Stator, in dem der Rotor drehbar gelagert ist,
- einer Detektoranordnung zum Erfassen von Röntgenstrahlung, die den Untersuchungsbereich unter verschiedenen Winkeln durchsetzt und zum Erzeugen von Detektorausgangssignalen,
- Mitteln zum Rekonstruieren der Absorptionsverteilung im Untersuchungsbereich aus den Detektorausgangssignalen und
- einer Ausgabeeinheit zur Darstellung der Absorptionsverteilung.

Ein derartiger Computertomograph ist aus der US-PS 4 190 772 und der US-PS 4 234 154 bekannt. Die Energieübertragung und die Datenübertragung erfolgen dabei über je einen Schleifring (sliping), und es ist damit möglich, eine Anzahl von Computertomogrammen unmittelbar nacheinander aufzunehmen, wobei die Zeit für die Aufnahme eines einzelnen Computertomogramms in der Größenordnung von einer Sekunde liegen kann. Bei Computertomographen, bei denen die Energie und die Daten vom Stator zum Rotor über Kabel übertragen werden, ist eine kontinuierliche Rotation nicht möglich. Der Rotor muß dabei in der Regel nach jeder Aufnahme eines Computertomogramms in eine Ausgangsstellung zurückgebracht und vor der nächsten Aufnahme wieder auf eine bestimmte Drehzahl hochgefahren werden. Der zeitliche Abstand zwischen zwei aufeinanderfolgenden Computertomogrammen ist daher bei derartigen Computertomographen wesentlich größer als bei solchen der eingangs genannten Art.

Datensipringe, die beim Computertomographen der eingangs genannten Art die Daten vom Rotor zum Stator übertragen, sind teuer bzw. wartungsaufwendig. Aufgabe der vorliegenden Erfindung ist es daher, einen Computertomographen der eingangs genannten Art so auszugestalten, daß er zwar ebenfalls kontinuierlich rotieren kann, einen Datensipring aber nicht benötigt. Diese Aufgabe wird erfindungsgemäß dadurch gelöst, daß die Mittel zur Rekonstruktion auf den Rotor angeordnet sind und daß eine Übertragungseinrichtung vorgesehen ist, die bei Stillstand des Rotors die die Absorptionsverteilung repräsentierenden Daten vom Rotor zum Stator überträgt.

Bei der Erfindung sind die Mittel zur Rekonstruktion auf dem Rotor angeordnet. Die Rekonstruktion kann stattfinden, während der Rotor rotiert, und die Übermittlung an die Ausgabeeinheit erfolgt bei Stillstand des Rotors. Die hierfür erforderliche Übertragungseinrichtung, beispielsweise eine steckbare Kabelverbindung, kann wesentlich einfacher sein als ein Datensipring, der in jeder Stellung des Rotors die Daten übertragen muß.

Es sei an dieser Stelle erwähnt, daß in der DE-PS 28 46 526, die sich ebenfalls auf einen kontinuierlich rotierenden Computertomographen bezieht, der Gedanke erörtert und verworfen wurde, anstelle eines Datensiprings einen Zwischenspeicher zu verwenden. Bei einem derartigen Computertomographen könnte die Rekonstruktion der Absorptionsverteilung anhand der im Zwischenspeicher gespeicherten Daten erst nach dem Stillstand des Rotors beginnen. Bei der Erfindung beginnt die Rekonstruktion schon während der Rotation und ist bei Stillstand des Rotors gegebenenfalls schon beendet, so daß danach nur noch die rekonstruierten Bilder der Absorptionsverteilung vom Rotor zu einer Ausgabeeinheit, z.B. einem Videomonitor, übertragen werden müssen.

Eine Weiterbildung der Erfindung sieht vor, daß die Übertragungseinrichtung eine optische Übertragungsstrecke umfaßt, die in einer definierten Winkelstellung des Rotors bzw. des Stators die Daten überträgt. Eine solche Übertragungsstrecke ist schneller und störsicherer als eine steckbare Kabelverbindung und kann wesentlich einfacher sein als ein optischer Datensipring zwischen Rotor und Stator. Sie erfordert lediglich einen Lichtsender (z.B. eine lichtemittierende Diode) und einen Lichtempfänger (Photodiode).

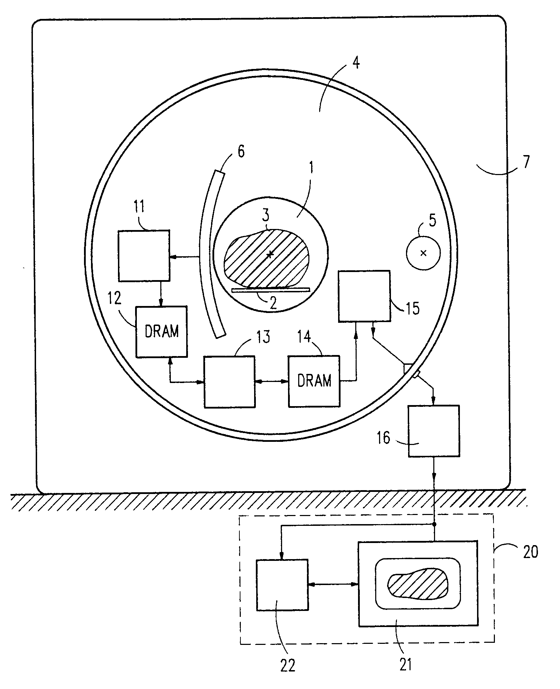

Die Erfindung wird nachstehend anhand der Zeichnung näher erläutert, die einen erfindungsgemäßen Computertomographen in schematischer Darstellung zeigt.

Der Computertomograph weist einen Untersuchungsbereich 1 auf, in dem sich auf einer Tischplatte 2 ein zu untersuchender Patient 3 befindet. Der Untersuchungsbereich 1 wird durch die zentrale Öffnung eines ringförmigen Rotors 4 definiert. Der Rotor ist in einem Rahmen 7 (Stator) drehbar gelagert und kann durch einen nicht näher dargestellten Antrieb für die Aufnahme von Tomogrammen in Drehung versetzt werden.

Alle Teile, die in der Zeichnung auf dem Rotor 4 dargestellt sind, rotieren während des Betriebes mit dem Rotor mit, während die übrigen Komponenten des Computertomographen nicht mitbewegt werden. Auf dem Rotor 4 befindet sich ein Röntgenstrahler 5, der ein fächerförmiges Strahlenbündel aussetzt, das den Untersuchungsbereich 1 durchsetzt und von einer Detektoranordnung 6 erfaßt wird, die jenseits des Untersuchungsbereichs 1 angeordnet ist, und die Intensität der Röntgenstrahlung auf verschiedenen Pfaden durch den Untersuchungsbereich mißt. In der Detektoranordnung werden die von den einzelnen Detektorzellen gelieferten Meßwerte verstärkt, digitalisiert und in eine Folge digitaler Datenworte umgesetzt, die den Detektorausgangssignalen entsprechen.

Die Rekonstruktionseinheit, die auf diesen Datenworten die Absorptionsverteilung rekonstruiert, ist erfindungsgemäß auf dem Rotor 4 angeordnet. Sie kann hard- und softwaremäßig den gleichen Aufbau haben wie die sonst bei Computertomographen üblichen, nicht mit dem Rotor mitbewegten Rekonstruktionseinheiten.

Die in der Zeichnung dargestellte Rekonstruktionseinheit weist eine Vorverarbeitungseinheit 11 auf, die von der Detektoranordnung 6 eintreffenden Daten in Echtzeit ("on the fly") vorverarbeitet und in einen Zwischenspeicher 12 schreibt. Die Vorverarbeitung kann ein Kalibrieren der Meßwerte, ein Prüfen der Meßwerte, das Verwerfen einzelner Meßwerte oder eines Satzes von Meßwerten usw. umfassen. Bei dem Zwischenspeicher 12 handelt es sich vorzugsweise um einen Halbleiterspeicher, beispielsweise ein wafer-DRAM..

Ein Prozessor 13 berechnet aus den im Zwischenspeicher 12 enthaltenen, durch die Vorverarbeitung erzeugten Rohdaten, die von der Absorption der Röntgenstrahlung längs eines Pfades durch den Untersuchungsbereich 1 abhängen, die Absorptionsverteilung, d.h. die Absorption an den einzelnen (Bild-) Punkten des Untersuchungsbereiches.

Auch diese Rekonstruktion kann bei einem hinreichend schnellen Prozessor nahezu in Echtzeit erfolgen. Die auf diese Weise rekonstruierte Absorptionsverteilung wird in einen Speicher 14 geschrieben, der vom gleichen Typ sein kann wie der Speicher 12. Vorzugsweise sind die in der Zeichnung getrennt dargestellten Speicher 12 und 14 Bereiche eines Speichers, der Rohdaten und rekonstruierte Daten gemeinsam speichert.

Auf dem Rotor 4 befindet sich weiterhin ein optischer Sender 15, der in einer definierten Winkelstellung des Rotors 4 in bezug auf den Stator 7 mit einem optischen Empfänger 16 auf dem Stator zusammenwirken kann. Sender bzw. Empfänger können beispielsweise eine lichtemittierende Diode bzw. eine Photodiode enthalten. Nach Abschluß der Messung der Absorptionsdaten wird der Rotor in die Position gebracht, in der Sender und Empfänger zusammenwirken, und die bis dahin rekonstruierten Bilddaten (bei einer Rekonstruktion in Echtzeit die Bilddaten sämtlicher Tomogramme) werden dann direkt auf einem Videomonitor 21 einer Arbeitsstation 20 wiedergegeben oder in einen Archivierungsspeicher 22 geschrieben. Wenn bei Stillstand die Bildrekonstruktion noch nicht abgeschlossen ist, können die Rohdaten aus dem Speicher (Bereich) 12 in einen entsprechenden Speicher der Arbeitsstation 20 oder direkt in den Archivierungsspeicher 22 übernommen werden. Die Rohdaten können aber auch durch die auf dem Rotor befindliche Elektronik weiterverarbeitet werden.

Die dazu erforderlichen Befehle sowie die Befehle an Röhre und Generator können entweder ebenfalls über eine optische Verbindung - jedoch mit einer geringen Übertragungsbandbreite - oder über einen mechanischen Schleifringkontakt (ggf. auch durch Modulation der zum Rotor übertragenen Versorgungsspannung) übertragen werden.

**Patentansprüche**

1. Computertomograph für kontinuierliche Rotation mit
   - einem einen Untersuchungsbereich (3) umschließenden Rotor (4),
   - einem Stator (7), in dem der Rotor drehbar gelagert ist,
   - einer Detektoranordnung (6) zum Erfassen von Röntgenstrahlung, die den Untersuchungsbereich unter verschiedenen Winkeln durchsetzt und zum Erzeugen von Detektorausgangssignalen,
   - Mitteln (11..14) zum Rekonstruieren der Absorptionsverteilung im Untersuchungsbereich aus den Detektorausgangssignalen und
   - einer Ausgabeeinheit (21) zur Darstellung der Absorptionsverteilung,

   dadurch gekennzeichnet, daß die Mittel (11..14) zur Rekonstruktion auf dem Rotor (4) angeordnet sind und daß eine Übertragungseinrichtung (15,16) vorgesehen ist, die bei Stillstand des Rotors die die Absorptionsverteilung repräsentierenden Daten vom Rotor zum Stator überträgt.

2. Computertomograph nach Anspruch 1, dadurch gekennzeichnet, daß die Übertragungseinrichtung eine optische Übertragungsstrecke (15,16) umfaßt, die in einer definierten Winkelstellung des Rotors (4) bzw. des Stators (7) die Daten überträgt.

| | **EINSCHLÄGIGE DOKUMENTE** | | EP 92201326.3 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl⁵) |
| D,A | <u>DE - A - 2 846 526</u><br>(SIEMENS AG)<br>   * Gesamt; besonders An-<br>     sprüche 1,2; Seite 4,<br>     erster Absatz *<br>       -- | 1,2 | G 01 T 1/29<br>A 61 B 6/03 |
| A | <u>EP - A - 0 286 678</u><br>(VOKOGAWA)<br>   * Gesamt; besonders Fig. 4 *<br>      -- | 1 | |
| D,A | <u>US - A - 4 190 772</u><br>(DINWIDDIE)<br>   * Gesamt *<br>      -- | 1 | |
| A | <u>DE - A - 3 538 035</u><br>(SIEMENS AG)<br>   * Gesamt *<br>      -- | 1 | |
| A | <u>US - A - 4 201 430</u><br>(DINWIDDIE)<br>   * Gesamt *<br>      ---- | 1 | |

**RECHERCHIERTE SACHGEBIETE (Int. Cl⁵)**

A 61 B   6/00
G 01 T   1/00
G 08 C 17/00
DATABASE WPI(L)
DATABASE JAPIO

Der vorliegende Recherchenbericht wurde für alle Patentansprüche erstellt.

| Recherchenort | Abschlußdatum der Recherche | Prüfer |
|---|---|---|
| WIEN | 26-08-1992 | WERNER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503 03 82